# EUROPEAN PATENT SPECIFICATION

(11) **EP 0 672 421 B1**
(45) Date of publication and mention of the grant of the patent: **20.03.2002**
(21) Application number: 94927840.2
(22) Date of filing: 04.10.1994
(51) Int. Cl.: A61K 38/17, C07K 14/72

(54) **ENDOTHELIN ACTIVITY INHIBITOR**
HEMMER DER AKTIVITÄT VON ENDOTHELIN
INHIBITEUR DE L'ACTIVITE DE L'ENDOTHELINE

(30) Priority: 07.10.1993 JP 25144593
(43) Date of publication of application: 20.09.1995
(73) Proprietor: Kurashiki Boseki Kabushiki Kaisha, Kurashiki-shi Okayama 710 (JP); Okada, Hidechika, Fukuoka-shi Fukuoka 814-01 (JP)
(72) Inventor: OHSHIMA, Kunihiro, Osaka-shi, Osaka 550 (JP); KAWAMURA, Seiko, Shijonawate-shi, Osaka 575 (JP); YAMAMOTO, Ryohei, Chita-shi, Aichi 478 (JP); NISHINO, Toyokazu, Ibaraki-shi, Osaka 567 (JP); OKADA, Hidechika, Fukuoka-shi, Fukuoka 814-01 (JP); BARANYI, Lajos, Toyohashi-shi, Aichi 441 (JP)
(74) Representative: von Kreisler, Alek, Dipl.-Chem.
(86) International application number: JP9401658
(87) International publication number: WO9509643

(56) References cited:
- EP-A- 0 522 868
- WO-A-86/05208
- LIN E.A.: "Cloning and functional expression of a vascular smooth muscle endothelin 1 receptor" PROCEEDINGS OF THE NATIONAL ACADEMY OF SCIENCES OF USA, vol. 88, April 1991, WASHINGTON US, pages 3185-3189, XP002062014
- ADACHI E.A.: "Cloning and characterization of cDNA encoding human A-type endothelin receptor" BIOCHEMICAL AND BIOPHYSICAL RESEARCH COMMUNICATIONS, vol. 180, no. 3, 14 November 1991, ORLANDO, FL US, pages 1265-1272, XP002062015
- YANAGISAWA E.A.: "A novel potent vasoconstrictor peptide produced by vascular endothelial cells" NATURE, vol. 332, 31 March 1988, LONDON GB, pages 411-415, XP002062016
- BARANYI E.A.: "The antisense homology box: a new motif within proteins that encodes biologically active peptides" NATURE MEDICINE., vol. 1, no. 9, September 1995, CO US, pages 894-901, XP002062017
- J. Biol. Chem., Vol. 268, No. 6 (1993), p. 3873-3879 (NABIL A. ELSHOURBAGY et al.).
- J. Biol. Chem., Vol. 267, No. 26 (1992), p. 18797-18804 (KIMINORI HOSODA et al.).

## Description

### Technical Field

The present invention relates to a pharmaceutical composition comprising a peptide with a high affinity to an endothelin.

### Background Art

Endothelin was first isolated from supernatant of cultured porcine aortic endothelial cells. When an endothelin precursor is converted to a mature peptide by enzymatic cleavage, it becomes a potent vasoconstrictor. This peptide is known to be the most potent agent for inducement of contraction of vascular smooth muscle and elevation of blood pressure in all existing substances, and is one of the bioactive substances which has recently come to the attention.

Endothelin consists of a family of three types of isopeptides, endothelin-1, endothelin-2, and endothelin-3. Each isopeptides consists of 21 amino acid residues. The structure of the endothelin-1 (ET-1) precursor has been described on the basis of a sequence obtained by cloning of a cDNA of ET-1 (see Nature, 332, 411-415 (1988)).

On the other hand, two types of endothelin receptors, i.e. A-type receptor which is specific to endothelin-1 and a nonspecific B-type receptor for the three types of the isopeptides, are known, and their amino acid sequences have been deduced from cloning of a cDNA respectively (see Biochem. Biophys. Res. Commun., 180, 1265-1272 (1991) and Biochem. Biophys. Res. Commun., 177, 34-39 (1991)). However, the binding sites for the endothelin or steric structures are unknown.

Endothelin is known to be synthesized in not only vascular endothelial cells but also many cells including nerve cells and tubular epithelial cells. It has been reported that in patients with hypertension, pulmonary hypertension, cerebral vasospasm following subarachnoid hemorrhage, renal failure, myocardial infarction, asthma, arteriosclerosis, vasculitis or sepsis, endothelin concentration in the blood elevate.

The study of substances specifically binding the endothelin and inhibiting its activity may be useful not only for the elucidation of the pathophysiological role of the endothelin but also for the development of the diagnosis and drug for treatment of above diseases.

Anti-ET-1 antibody is known as a substance which have an affinity specific to the endothelin and directly inhibits its activity. In general, the amount of endothelin has been measured by RIA or EIA method using this antibody. This antibody has been reported to be used to directly modify or inhibit the endothelin activity in many organs (Japanese Non-examined Patent Publication No.2-238894). With the exception of the anti-ET-1 antibody, however, substance having an affinity specific to the endothelin has not been obtained yet.

It was reported when DNA or RNA fragment was complementary to DNA or mRNA encoding for a target peptide (peptide I), a peptide II having an amino acid sequence deduced from the codons on this fragment and aligned according to the sequence of the fragment, had an affinity to the original peptide I, in angiotensin (Proc. Natl. Acad. Sci. USA, 85, 2518-2522 (1988)), luteinizing hormone releasing hormone (Proc. Natl. Acad. Sci. USA, 83, 9714-9718 (1986)), and adrenocorticotropic hormone (Proc. Natl. Acad. Sci. USA, 82, 1372-1375 (1985)), etc. On the basis of these findings, a method for obtaining a complementary peptide defined according to the sequence of non-reading DNA or complementary RNA was disclosed by Blalock in Japanese Non-examined Patent Publication No.62-502513.

### Problems of Prior Art

Because an antibody is a biosynthesized protein, there are several problems including its storage stability, purification cost, and an alteration of the antibody molecule caused by an alteration of animals or subculturing cells by which the antibody is produced. These problems disturb stable supply of the antibody. In addition, for in vivo administration of the antibody, there are problems including immunogenicity, and reduction in the activity due to a digestive enzyme.

The development of a low-molecular substance with a high affinity to the endothelin is desired and an effort to search such a substance has been made in the art. However, main source, such as supernatant of cultured soil bacteria, and the strategy for searching the candidate substance depend on an accidental occurrence.

On the other hand, a peptide having an amino acid sequence deduced from DNA or RNA sequence which is complementary to cDNA or mRNA encoding for a target peptide, do not always show the aimed affinity to the target peptide (see Biochem. J., 261, 310-311 (1989)).

It is the object of the present invention to provide a low molecular peptide which have a high affinity to endothelin and inhibit its activity.

### Disclosure of the Invention

To solve the above problems, on the basis of the information about both amino acid sequences of an endothelin precursor and an endothelin receptor, a molecule was designed to obtain a peptide, which has a higher affinity to the endothelin with high probability, and a group of peptides inhibiting the activity of endothelin were obtained.
Thus, the present relates to
(1) a pharmaceutical composition comprising an endothelin activity inhibitor comprising a peptide including such a part of an amino acid sequence of a human endothelin receptor corresponding to a region wherein at least 80 % of the amino acid sequence is complementary to each other when the amino acid sequence estimated on the basis of a cDNA of the human endothelin receptor is compared with the one estimated on the basis of a cDNA of a human endothelin precursor, and including the following amino acid sequence: ALSVD(X)Y(X)AVAS where X represents any amino acid,
   or a conjugate thereof with a polymer or a protein;
(2) a A pharmaceutical composition comprising an endothelin activity inhibitor comprising a peptide including such a part of an amino acid sequence of a human endothelin receptor corresponding to a region wherein at least 80 % of the amino acid sequence is complementary to each other when the amino acid sequence estimated on the basis of a cDNA of the human endothelin receptor is compared with the one estimated on the basis of a cDNA of a human endothelin precursor, and including one of the following amino acid sequences (a) to (d): 
(a) QG IGIPLITAIEI
(b) IAD NAERYSANLSSHV
(c) F LGTTLRPPNLALP
(d) L NRRNGSLRIALSEHLKNRREVA
 or a conjugate thereof with a polymer or a protein; and
(3) an endothelin activity inhibitor comprising a peptide including an amino acid sequence selected from: 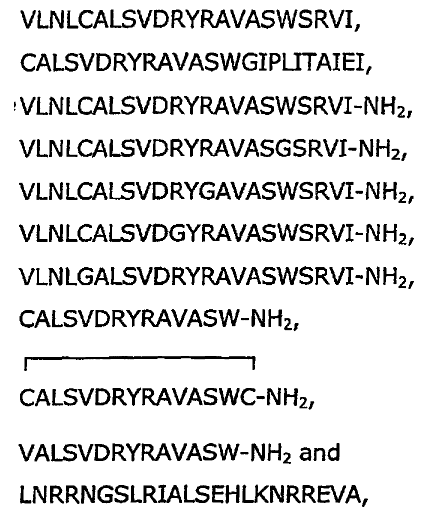
VLNLCALSVDRYRAVASWSRVI,
CALSVDRYRAVASWGIPLITAIEI,
VLNLCALSVDRYRAVASWSRVI-NH2,
VLNLCALSVDRYRAVASGSRVI-NH2,
VLNLCALSVDRYGAVASWSRVI-NH2,
VLNLCALSVDGYRAVASWSRVI-NH2,
VLNLGALSVDRYRAVASWSRVI-NH2,
CALSVDRYRAVASW-NH2,
CALSVDRYRAVASWC-NH2,
VALSVDRYRAVASW-NH2 and
LNRRNGSLRIALSEHLKNRREVA,
 or a conjugate thereof with a polymer or a protein.

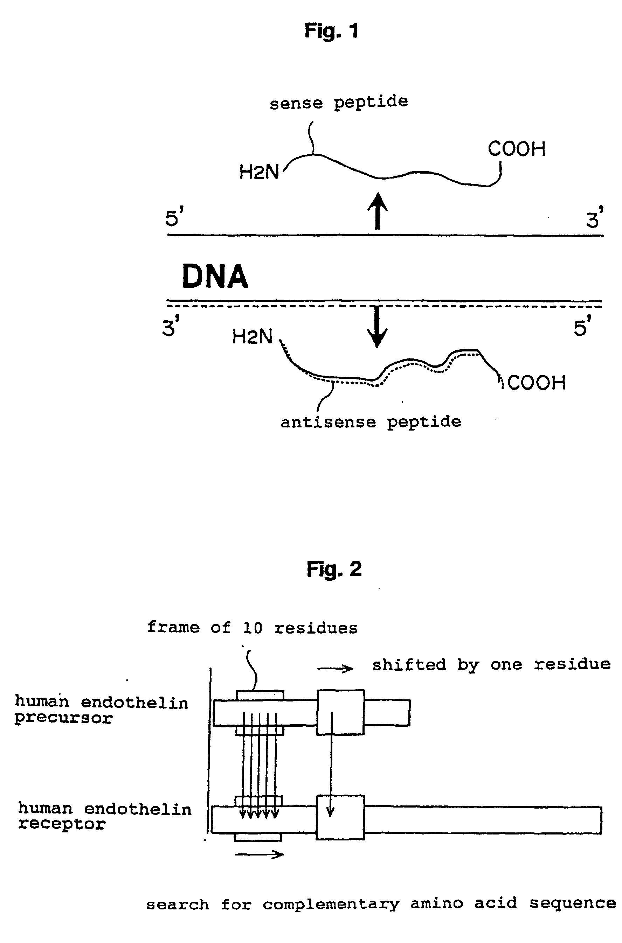

A complementary peptide is generally an anti-sense peptide consisting of an amino acid sequence coded by a DNA sequence as opposed to a cDNA encoding for a bioactive sense peptide, as illustrated in Figure 1. In this application, amino acid complementicy is evaluated by comparing each amino acid residues, in which, for example, when there are several codons which encode for an amino acid A, all amino acids coded by the respective anti-codons are referred to be "complementary" to amino acid A. The complementary amino acids for each amino acid are shown in the following Table 1. In this specification, amino acid residues are described by one letter abbreviation which is generally used in the art. In addition, unless otherwise noted, amino acids are L-amino acids.

**Table 1:**

| Complementary amino acids | | | |
|---|---|---|---|
| Amino acid | Complementary amino acid(s) | Amino acid | Complementary amino acid(s) |
| I | N, D, Y | P | G, W, R |
| V | N, D, H, Y, | A | G, S, C, R |
| L | K, E, Q | T | G, S, C, R |
| F | K, E | Q | L |
| M | H | E | L, F |
| W | P | K | L, F |
| R | P, S, T, A | N | I, V |
| G | P, S, T, A | D | I, V |
| C | T, A | Y | I, V |
| S | G, T, A, R | H | V, M |

The amino acid sequence of the human endothelin precursor was determined by cloning of a cDNA of human endothelin receptor-1 (Nature, 332, 411-415 (1988)). On the other hand, it is known that there are two types of human endothelin receptors: i.e. A-type receptor and B-type receptor, and their cDNAs have been cloned, however, their active sites and steric structures are still unknown. In the molecular design of the peptide of the present invention, the amino acid sequence deduced from the cDNA encoding for endothelin-1 specific A-type receptor (see Biochem. Biophys. Res. Commun., 180, 1265-1272 (1991)) was used.

In the present invention, the amino acid sequences between the human endothelin precursor and the human endothelin receptor are compared as the manner described below, whereby a portion of the amino acid sequence of human endothelin receptor, in which 80% of amino acid sequence is complementary between the both can be obtained. First, a frame of 10 residues from the N-terminal, that is amino acid 1-10, on the amino acid sequence of the endothelin precursor, is compared with the above whole amino acid sequence of the endothelin receptor, and then a region in which more than eight amino acids are complementary to the above frame is selected from the sequence of the endothelin receptor according to the Table 1 (see Figure 2). Subsequently, the frame on the precursor of endothelin is shifted by one residue to the C-terminal, then the frame of next 10 residues, that is amino acid 2-11, is compared with the whole sequence of the endothelin receptor, whereby a region which more than eight amino acids are complementary to the 2nd frame is selected from the amino acid sequence of the endothelin receptor according to the Table 1. The same procedures are repeated until the frame moves over the whole sequence of the precursor, and whereby regions which more than 80% of amino acid sequence is complementary to each other, according to the Table 1, are selected from the amino acid sequence of endothelin receptor.

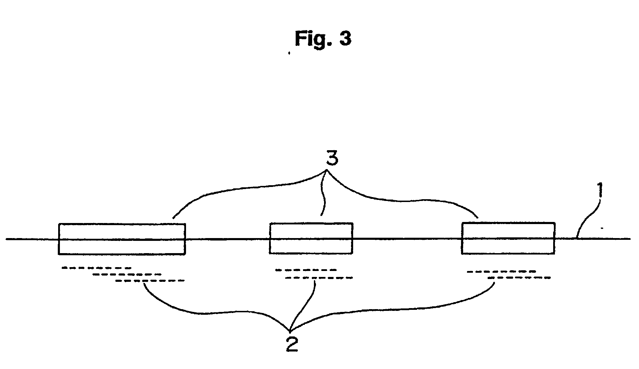

In the present invention, the part of the amino acid sequence of the endothelin receptor where at least 80% of the amino acid sequence is complementary to that of endothelin precursor, include both a portion which shows complementary to only one frame of the endothelin precursor, and a portion which shows complementary to several frames of the precursor as shown in Figure 3. The peptides containing such amino acid sequences are expected to have high affinity to endothelin.

The preferred amino acid sequences obtained by the above method are as follows. The number represents the corresponding sequence ID number of the sequence listing.

C1: VLNLCALSVDRYRAVASWSRVI

C2: CALSVDRYRAVASWGIPLITAIEI

C3: QGIGIPLITAIEI

C4: IADNAERYSANLSSHV

C5: FLGTTLRPPNLALP

C6: LNRRNGSLRIALSEHLKNRREVA.

The peptides comprised in the pharmaceutical composition according to the present invention include analogues of the above peptide C1 having the following sequence: ALSVD(X)Y(X)AVASW wherein X represents any amino acid. These analogues also show an inhibitory effect against the activity of endothelin.

Preferred peptides include a peptide in which carboxyl group of the C-terminal of the C1 peptide is amidaded, and peptides in which any one of the amino acid residues of above peptide having amidaded C-terminal is substituted for glycine. Concretely preferred peptides are as follows:

C11: VLNLCALSVDRYRAVASWSRVI-NH2
C12: VLNLCALSVDRYRAVASGSRVI-NH2
C13: VLNLCALSVDRYGAVASWSRVI-NH2
C14: VLNLCALSVDGYRAVASWSRVI-NH2
C15: VLNLGALSVDRYRAVASWSRVI-NH2.

In the above and below formulae, "-NH₂" represents that carboxyl group of C-terminal is converted to be an amide group.

In addition, a peptide which amino acid residues at N and/or C-terminal of the C1 peptide are removed and/or some residues are partially substituted is also preferably used. Concretely preferred sequence are as follows:

C16: ALSVDRYRAVASW-NH2

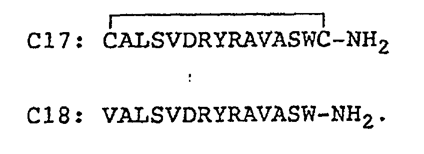
C17: CALSVDRYRAVASWC-NH2
:
C18: VALSVDRYRAVASW-NH2.

### Brief Description of Drawings

Figure 1 is a schematic illustration of a complementary peptide.
Figure 2 is a schematic illustration of a method for searching an amino acid sequence of a peptide of the present invention.
Figure 3 is a schematic illustration of a portion of endothelin receptor which is complementary to the endothelin precursor.
Figure 4 is a graph showing change of intracellular calcium concentration induced by endothelin-1.
Figure 5 is a graph showing the inhibitory effect of peptides C1, C2, C3, and C5 of the present invention on endothelin-1 induced increase of intracellular calcium concentration.
Figure 6 is a graph showing the inhibitory effect of peptides C3-MAP, C4-MAP, and C6-MAP of the present invention on endothelin-1 induced increase of intracellular calcium concentration.
Figure 7 is a graph showing the inhibitory effect of peptide C1 of the present invention on endothelin-1 induced increase of intracellular calcium concentration.
Figure 8 is a graph showing the inhibitory effect of peptide C1-MAP of the present invention on endothelin-1 induced increase of intracellular calcium concentration.
Figure 9 is a graph showing the inhibitory effect of peptide C3-MAP of the present invention on endothelin-1 induced increase of intracellular calcium concentration.
Figure 10 is a graph showing the inhibitory effect of peptides C1 to C6 of the present invention on the endothelin-1 induced contraction of vascular smooth muscle.
Figure 11 is a graph showing the inhibitory effect of peptides C1-MAP to C6-MAP of the present invention on the endothelin-1 induced contraction of vascular smooth muscle.
Figure 12 is a graph showing the inhibitory effect of peptides C11 to C18 of the present invention on the endothelin-1 induced contraction of vascular smooth muscle.

### Description of Symbols

1: The amino acid sequence of human endothelin receptor, 2: a frame of amino acid sequence of human endothelin precursor which more than 80% of the amino acid sequence is complementary to that of human endothelin receptor, 3: an amino acid sequence contained in the peptide of the present invention

### Mode for Carrying Out the Invention

The endothelin activity inhibitor preferably includes amino acid sequence of at least any one of the above C1 to C6 and C11 to C18 peptides. The preferred peptide may be comprised from the above defined amino acid sequence alone or a polypeptide including a repeated portion of above amino acid sequence.

When the peptide is used as a drug or diagnostic agent, a polymer or a protein may be used as a carriers to improve its effect and properties. The polymer includes latex particles, agarose resin, acrylamide resin, polystyrene resin, polyethylene resin, and poly-L-lysine. The protein includes human albumin, bovine serum albumin, Keyhole Limpet hemocyanin and bovine thyroglobulin.

The peptide can be synthesized according to a standard method. The preferred method for synthesizing the peptide includes chemical synthesis such as solid and solution phase methods. In addition, a DNA vector including a sequence encoding for the peptide may be constructed and cultured cells or bacteria may be transformed with the vector, and then peptide may be produced by above transformants.

### Examples

The present invention is further illustrated by the following examples:

### Synthesis of peptides

Peptides of C1 to C6 and C11 to C18 described below were synthesized.

C1: VLNLCALSVDRYRAVASWSRVI
C2: CALSVDRYRAVASWGIPLITAIEI
C3: QGIGIPLITAIEI
C4: IADNAERYSANLSSHV
C5: FLGTTLRPPNLALP
C6: LNRRNGSLRIALSEHLKNRREVA
C11: VLNLCALSVDRYRAVASWSRVI-NH2
C12: VLNLCALSVDRYRAVASGSRVI-NH2
C13: VLNLCALSVDRYGAVASWSRVI-NH2
C14: VLNLCALSVDGYRAVASWSRVI-NH2
C15: VLNLGALSVDRYRAVASWSRVI-NH2

C16: CALSVDRYRAVASW-NH2
C17: CALSVDRYRAVASWC-NH2
C18: VALSVDRYRAVASW-NH2

Peptides C1 to C18 were synthesized by solid phase method using a peptide synthesizer (PEPTICOUPLER 2200: VEGA Corp.). The peptides with a carboxyl terminal were synthesized using PAM resin, and the peptides with amidaded C-terminal were synthesized using MBHA resin. The obtained peptide-resin was treated with anhydrous hydrogen fluoride whereby the peptide was cleaved from the resin and deprotected, then the resulted peptide was extracted with acetic acid-acetonitrile-water (1:2:2) and was purified by high-performance liquid chromatography. The obtained peptide was confirmed by amino acid analysis.

### Synthesis of polypeptides having poly-L-lysine at their C-terminal

Peptides C1-MAP to C6-MAP described below were synthesized.

C1-MAP: (VLNLCALSVDRYRAVASWSRVI)8K4K2K-β-Ala-OH
C2-MAP: (CALSVDRYRAVASWGIPLITAIEI)8K4K2K-β-Ala-OH
C3-MAP: (QGIGIPLITAIEI)8K4K2K-β-Ala-OH

C4-MAP: (IADNAERYSANLSSHV)₈K₄K₂K-β-Ala-OH

C5-MAP: (FLGTTLRPPNLALP)₈K₄K₂K-β-Ala-OH

C6-MAP: (LNRRNGSLRIALSEHLKNRREVA)₈K₄K₂K-β-Ala-OH

The synthesis was performed by solid phase method using a peptide synthesizer (PEPTICOUPLER 2200). The obtained peptide-resin was treated with anhydrous hydrogen fluoride whereby the peptide was cleaved from the resin and deprotected, and after extraction with 0.1M Tris-HCl (pH8.0) containing 8M urea, the peptide was purified by dialysis. The obtained peptide was confirmed by amino acid analysis.

### Inhibition of endothelin-induced increase of intracellular calcium ion concentration

It is known that addition of endothelin into culture cell causes the elevation of intracellular Ca²⁺ concentration. Using mouse 3T3 cells, the inhibitory effect of the peptide of the present invention on the activity of endothelin was examined by changes in the intracellular calcium ion concentration.

### 1. Measurement of intracellular calcium ion concentration

Commercially available 3T3 cells (ATCC No.CCL92) cultured in a flask according to the standard method were used in the experiments. The cells adhered to a inner wall of the flask were harvested by trypsinization treatment, washed three times with Ca²⁺-free phosphate buffered saline (PBS) and suspended into 200 µl of the Ca²⁺-free PBS. Into the cell suspension, a fluorescent indicator of Ca²⁺, FLUO-3AM (Wako chemicals: 1 mM in DMSO solution), was added at a final concentration of 1 µM. The cells were incubated at 37°C for 10 minutes to allow them to uptake the indicator. Subsequently, the cells were washed three times with Ca²⁺-free PBS, and suspended in buffer A (140 mM NaCl, 4 mM KCl, 1 mM Na₂HPO₄, 1 mM MgCl₂, 1.25 mM CaCl₂, 11 mM glucose, 5 mM HEPES (pH7.4) containing 0.2% bovine serum albumin), to about 10⁵ cells/ml.

The increase in relative fluorescence intensity caused by Ca²⁺ influx was measured with FACScan of Becton, Dickinson & Co.. In the measurement, a base line was determined over 20 seconds, then a test substance (for example endothelin) was added into the sample and followed the time course of the relative fluorescence intensity for about 80 seconds.

### 2. Increase of intracellular calcium ion concentration by addition of endothelin

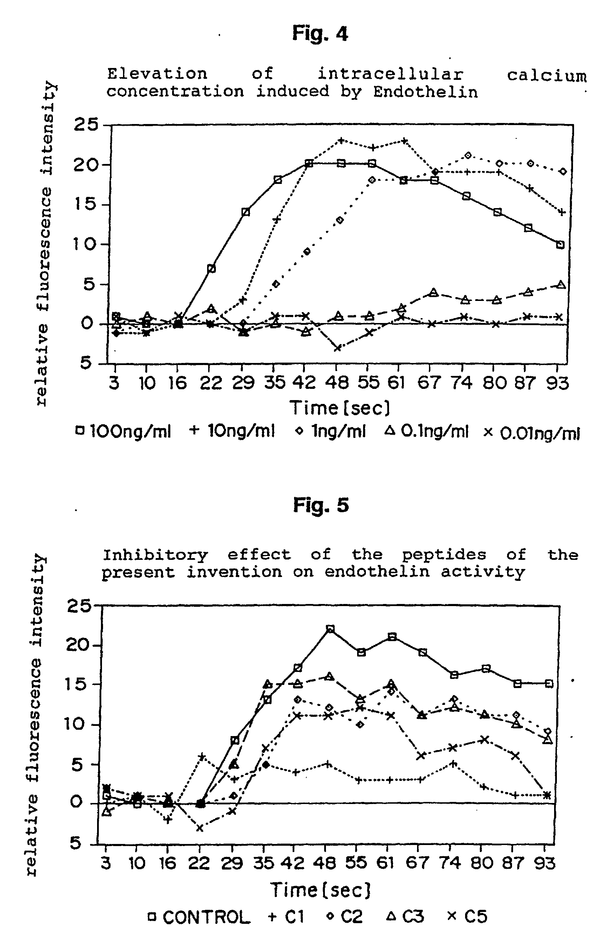

Into each aliquots of the above cell suspension, endothelin-1 was added at a final concentration of 0.01 to 100 ng/ml to observe the changes in the relative fluorescence intensity. As shown in Figure 4, the concentration of Ca²⁺ was more extremely increased as the amount of endothelin increased, indicating that the increase of the intracellular calcium ion concentration occurs in proportion to the concentration of endothelin.

### 3. Inhibition of increase of intracellular calcium ion concentration by the peptides (I)

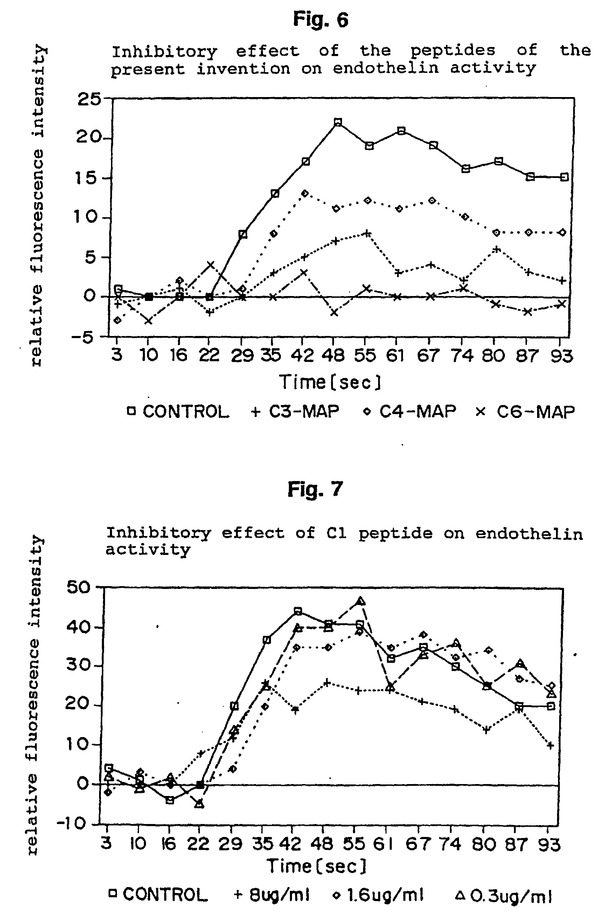

The peptides were added into the aliquots of the cell suspension containing the fluorescent indicator at a final concentration of 200 µg/ml respectively, and preincubated at 25°C for 20 minutes. To the respective suspension, endothelin-1 was added at a final concentration of 10 ng/ml in a similar manner to the above experiment, and changes in the relative fluorescence intensity were examined for 80 seconds. The results are shown in Figures 5 and 6.

Figures 5 and 6 show that peptides C1, C2, C3, C5, C3-MAP, C4-MAP, and C6-MAP inhibit the endothelin-1 induced increase of intracellular calcium ion concentration respectively.

### 4. Inhibition of increase of intracellular calcium ion concentration by the peptides (II)

To aliquots of dimethylsulfoxide (DMSO) solution of endothelin-1 (10 µg/ml), each peptide was added at final concentrations of 200, 40, 8, 1.6, 0.3 µg/ml respectively, and the mixtures were preincubated at 25°C for 20 minutes.

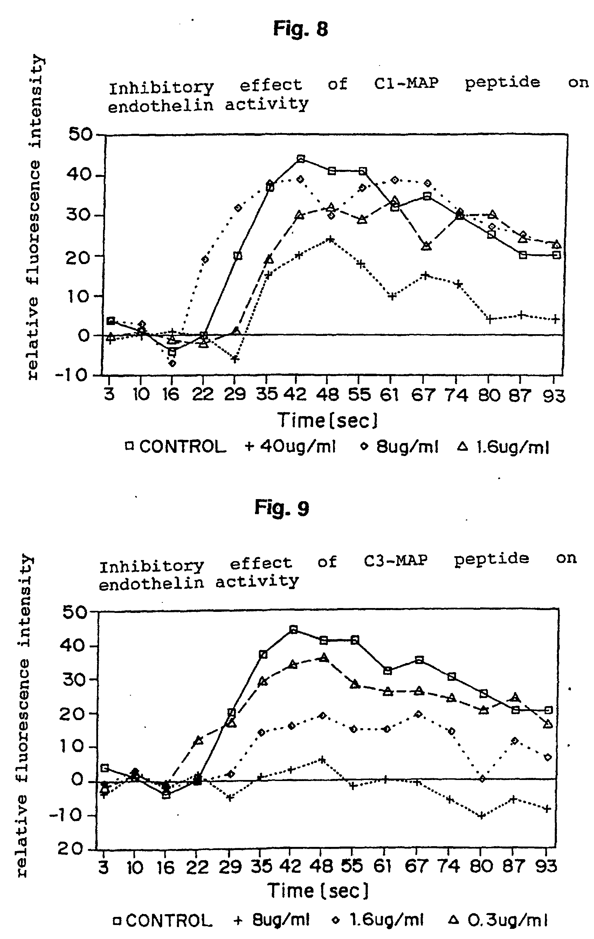

The mixtures of the endothelin and the peptide were added into the aliquots of the cell suspension at a endothelin concentration of 10 ng/ml respectively, and then changes in the relative fluorescence intensity were examined for 80 seconds. The results are shown in Figures 7 to 9.

These Figures suggest that peptides C1, C1-MAP, and C3-MAP inhibit the endothelin induced increase of intracellular calcium ion concentration respectively.

### Effect of the peptides on endothelin induced vascular contraction

Endothelin is known to contract the vascular smooth muscle. Using rat femoral arteries, the effect of the peptide of the present invention on the endothelin induced contraction of vascular smooth muscle was examined.

The femoral arteries of male Wister rats (body weight of 220-350 g, 7-9 weeks old) were isolated and cut into spiral strips with length of 20 mm and width of 1.4mm. The obtained strips were set to isometric transducers, and sunk into 20 ml of Krebs-Henseleit Bicarbonate (KHB) buffer (NaCl 1150 mM; KCl 4.7 mM; CaCl 2.5 mM; MgCl₂ 1.2 mM; NaHCO₃ 25.0 mM; KH₂PO₄ 1.2 mM; dextrose 10.0 mM, pH7.5, 37°C, bubbled with 95%O₂ and 5%CO₂). The strips were mounted vertically under an optimal resting tension of 500 mg. The ability of the contraction of the strips were confirmed by adding KCl into the buffer at a final concentration of 40 mM, and then the confirmed strips were used after washing with the buffer.

First, endothelin-1 was added to the buffer at a final concentration of 10⁻⁹M. When the contraction became stable, the peptide of the present invention was accumulatively added, and vascular relaxation was measured. Ultimately, papaverine, a known potent smooth muscle relaxant, was added at a final concentration of 10⁻⁴M to obtain the maximum relaxation which was expressed as 100%, and estimated relaxation at each concentration of the peptides.

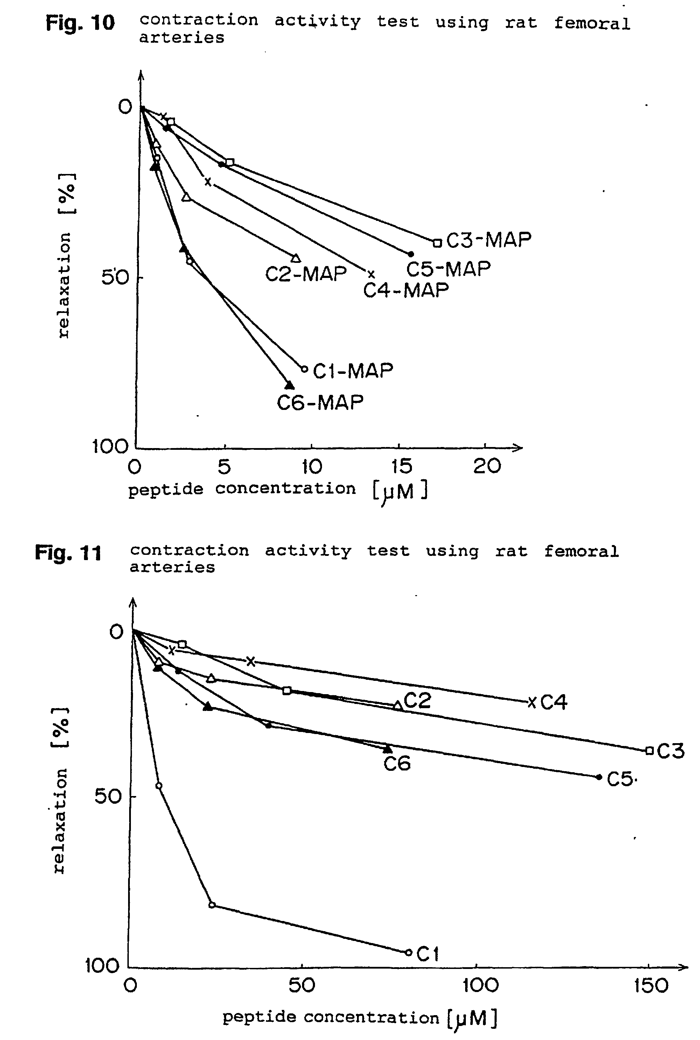

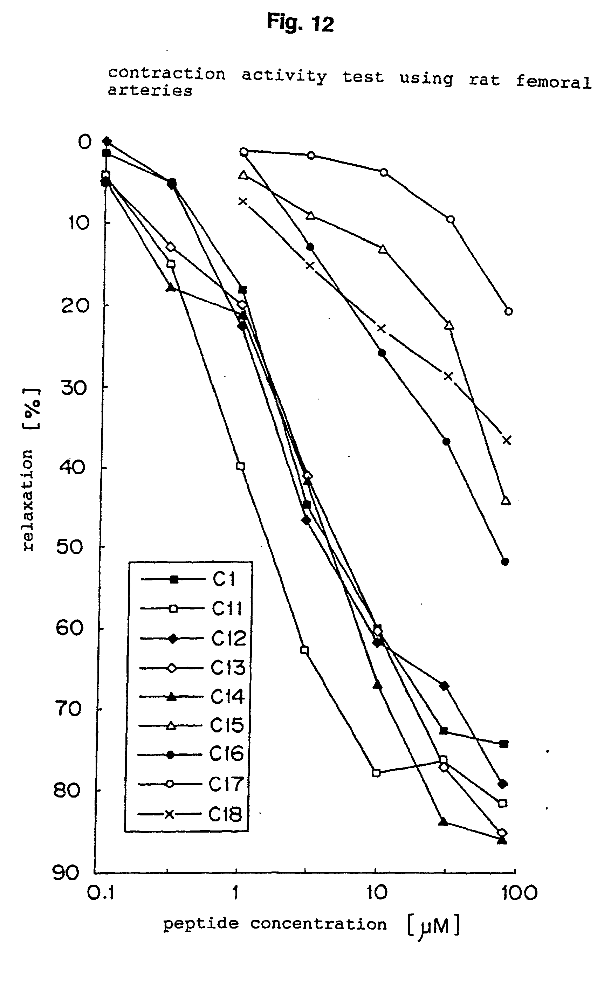

The results are shown in Figures 10 - 12. It is noted that the peptides show potent relaxation effect on endothelin induced vascular smooth muscle- contraction.

### Industrial Applicability

The peptides can be chemically synthesized, so that it can be provided at a low cost as compared with an antibody, and the endothelin activity inhibitor can be provided at a stable quality. In addition, because of the relatively low molecular weight, the inhibitor can be used not only as a reagent for detecting endothelin but also as a remedy for endothelin-related various diseases.

## Claims

1. A pharmaceutical composition comprising an endothelin activity inhibitor comprising a peptide
- including such a part of an amino acid sequence of a human endothelin receptor corresponding to a region wherein at least 80 % of the amino acid sequence is complementary to each other when the amino acid sequence estimated on the basis of a cDNA of the human endothelin receptor is compared with the one estimated on the basis of a cDNA of a human endothelin precursor, and
- including the following amino acid sequence: ALSVD(X)Y(X)AVAS
where X represents any amino acid,
or a conjugate thereof with a polymer or a protein.

2. The pharmaceutical composition of claim 1, in which the peptide includes the following amino acid seqence: VLNLCALSVDRYRAVASWSRVI or a conjugate thereof with a polymer or a protein.

3. The pharmaceutical composition of claim 1, in which the peptide includes the following amino acid seqence: CALSVDRYRAVASWGIPLITAIEI or a conjugate thereof with a polymer or a protein.

4. The pharmaceutical composition of claim 1; in which the peptide includes the following amino acid seqence: VLNLCALSVDRYRAVASWSRVI-NH2 or a conjugate thereof with a polymer or a protein.

5. The pharmaceutical composition of claim 1, in which the peptide includes the following amino acid seqence: VLNLCALSVDRYRAVASGSRVI-NH2 or a conjugate thereof with a polymer or a protein.

6. The pharmaceutical composition of claim 1, in which the peptide includes the following amino acid seqence: VLNLCALSVDRYGAVASWSRVI-NH2 or a conjugate thereof with a polymer or a protein.

7. The pharmaceutical composition of claim 1, in which the peptide includes the following amino acid seqence: VLNLCALSVDGYRAVASWSRVI-NH2 or a conjugate thereof with a polymer or a protein.

8. The pharmaceutical composition of claim 1, in which the peptide includes the following amino acid seqence: VLNLGALSVDRYRAVASWSRVI-NH2 or a conjugate thereof with a polymer or a protein.

9. The pharmaceutical composition of claim 1, in which the peptide includes the following amino acid seqence: CALSVDRYRAVASW-NH2 or a conjugate thereof with a polymer or a protein.

10. The pharmaceutical composition of claim 1, in which the peptide includes the following amino acid seqence: 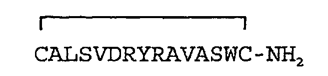
CALSVDRYRAVASWC-NH2
 or a conjugate thereof with a polymer or a protein.

11. The pharmaceutical composition of claim 1, in which the peptide includes the following amino acid seqence: VALSVDRYRAVASW-NH₂ or a conjugate thereof with a polymer or a protein.

12. A pharmaceutical composition comprising an endothelin activity inhibitor comprising a peptide
- including such a part of an amino acid sequence of a human endothelin receptor corresponding to a region wherein at least 80 % of the amino acid sequence is complementary to each other when the amino acid sequence estimated on the basis of a cDNA of the human endothelin receptor is compared with the one estimated on the basis of a cDNA of a human endothelin precursor, and
- including one of the following amino acid sequences (a) to (d): 
(a) QGIGIPLITAIEI
(b) IADNAERYSANLSSHV
(c) FLGTTLRPPNLALP
(d) LNRRNGSLRIALSEHLKNRREVA
 or a conjugate thereof with a polymer or a protein.

13. An endothelin activity inhibitor comprising a peptide including an amino acid sequence selected from: 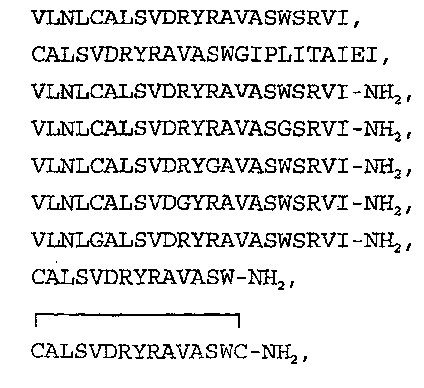
VLNLCALSVDRYRAVASWSRVI,
CALSVDRYRAVASWGIPLITAIEI,
VLNLCALSVDRYRAVASWSRVI-NH2,
VLNLCALSVDRYRAVASGSRVI-NH2,
VLNLCALSVDRYGAVASWSRVI-NH2,
VLNLCALSVDGYRAVASWSRVI-NH2,
VLNLGALSVDRYRAVASWSRVI-NH2,
CALSVDRYRAVASW-NH2,
CALSVDRYRAVASWC-NH2,
 
VALSVDRYRAVASW-NH2 and
LNRRNGSLRIALSEHLKNRREVA,
 or a conjugate thereof with a polymer or a protein.

## Patentansprüche

1. Pharmazeutische Zusammensetzung, die einen Inhibitor der Endothelinaktivität umfasst, der folgendes umfasst: ein Peptid, das
- einen solchen Teil einer Aminosäuresequenz eines humanen Endothelin-Rezeptors enthält, der einem Bereich entspricht, in dem wenigstens 80% der Aminosäuresequenz komplementär zueinander ist, wenn die auf der Basis einer cDNA des humanen Endothelin-Rezeptors abgeschätzte Aminosäuresequenz mit einer auf der Basis einer cDNA des humanen Endothelln-Vorläufers abgeschätzten Sequenz verglichen wird; und
- die folgende Aminosäureseguenz enthält: ALSVD(X)Y(X)AVAS
wobei X irgendeine Aminosäure darstellt;
oder ein Konjugat davon mit einem Polymer oder einem Protein.

2. Pharmazeutische Zusammensetzung gemäß Anspruch 1, wobei das Peptid die folgende Aminosäuresequenz VLNLCALSVDRYRAVASWSRVI oder ein Konjugat davon mit einem Polymer oder einem Protein enthält.

3. Pharmazeutische Zusammensetzung gemäß Anspruch 1, wobei das Peptid die folgende Aminosäuresequenz CALSVDRYRAVASWGIPLITAIEI oder ein Konjugat davon mit einem Polymer oder einem Protein enthält.

4. Pharmazeutische Zusammensetzung gemäß Anspruch 1, wobei das Peptid die folgende Aminosäuresequenz VLNLCALSVDRYRAVASWSRVI-NH2 oder ein Konjugat davon mit einem Polymer oder einem Protein enthält.

5. Pharmazeutische Zusammensetzung gemäß Anspruch 1, wobei das Peptid die folgende Aminosäuresequenz VLNLCALSVDRYRAVASGSRVI-NH2 oder ein Konjugat davon mit einem Polymer oder einem Protein enthält.

6. Pharmazeutische Zusammensetzung gemäß Anspruch 1, wobei das Peptid die folgende Aminosäuresequenz VLNLCALSVDRYGAVASWSRVI-NH2 oder ein Konjugat davon mit einem Polymer oder einem Protein enthält.

7. Pharmazeutische Zusammensetzung gemäß Anspruch 1, wobei das Peptid die folgende Aminosäuresequenz VLNLCALSVDGYRAVASWSRVI-NH2 oder ein Konjugat davon mit einem Polymer oder einem Protein enthält.

8. Pharmazeutische Zusammensetzung gemäß Anspruch 1, wobei das Peptid die folgende Aminosäuresequenz VLNLGALSVDRYRAVASWSRVI-NH2 oder ein Konjugat davon mit einem Polymer oder einem Protein enthält.

9. Pharmazeutische Zusammensetzung gemäß Anspruch 1, wobei das Peptid die folgende Aminosäuresequenz CALSVDRYRAVASW-NH2 oder ein Konjugat davon mit einem Polymer oder einem Protein enthält.

10. Pharmazeutische Zusammensetzung gemäß Anspruch 1, wobei das Peptid die folgende Aminosäuresequenz CALSVDRYRAVASWC-NH2 oder ein Konjugat davon mit einem Polymer oder einem Protein enthält.

11. Pharmazeutische Zusammensetzung gemäß Anspruch 1, wobei das Peptid die folgende Aminosäuresequenz VALSVDRYRAVASW-NH2 oder ein Konjugat davon mit einem Polymer oder einem Protein enthält.

12. Pharmazeutische Zusammensetzung, die einen Inhibitor der Endothelinaktivität umfasst, der folgendes umfasst: ein Peptid, das
- einen solchen Teil einer Aminosäuresequenz eines humanen Endothelin-Rezeptors enthält, der einem Bereich entspricht, in dem wenigstens 80% der Aminosäuresequenz komplementär zueinander ist, wenn die auf der Basis einer cDNA des humanen Endothelin-Rezeptors abgeschätzte Aminosäuresequenz mit einer auf der Basis einer cDNA des humanen Endotheiin-Vorläufers abgeschätzten Sequenz verglichen wird; und
- eine der folgenden Aminosäuresequenzen (a) bis (d) enthält: 
(a) QGIGIPLITAIEI
(b) IADNAERYSANLSSHV
(c) FLGTTLRPPNLALP
(d) LNRRNGSLRIALSEHLKNRREVA
 oder ein Konjugat davon mit einem Polymer oder einem Protein.

13. Inhibitor der Endothelinaktivität, der folgendes umfasst: ein Peptid, das eine Aminosäuresequenz enthält, die ausgewählt ist aus: 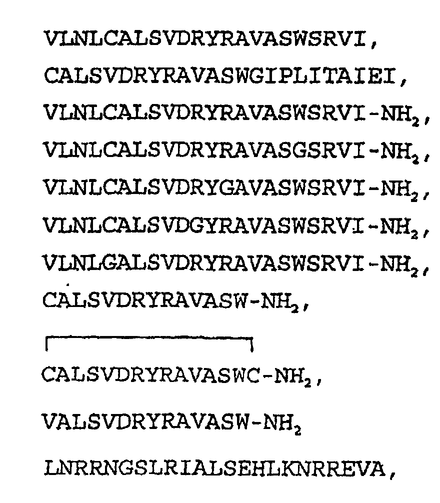
VLNLCALSVDRYRAVASWSRVI,
CALSVDRYRAVASWGIPLITAIEI,
VLNLCALSVDRYRAVASWSRVI-NH2,
VLNLCALSVDRYRAVASGSRVI-NH2,
VLNLCALSVDRYGAVASWSRVI-NH2,
VLNLCALSVDGYRAVASWSRVI-NH2,
VLNLGALSVDRYRAVASWSRVI-NH2,
CALSVDRYRAVASW-NH2,
CALSVDRYRAVASWC-NH2,
VALSVDRYRAVASW-NH2
LNRRNGSLRIALSEHLKNRREVA,
 oder ein Konjugat davon mit einem Polymer oder einem Protein.

## Revendications

1. Composition pharmaceutique comprenant un inhibiteur de l'activité de l'endothéline comprenant un peptide
- incluant une partie de la séquence d'acides aminés d'un récepteur humain de l'endothéline telle qu'elle corresponde à une région avec au moins 80 % de complémentarité entre les séquences d'acides aminés, quand on compare la séquence d'acides aminés estimée sur la base d'un ADNc du récepteur humain de l'endothéline à celle estimée sur la base d'un ADNc d'un précurseur humain de l'endothéline et
- incluant la séquence d'acides aminés suivante : ALSVD(X)Y(X)AVAS
dans laquelle X représente un acide aminé quelconque,
ou un de ses conjugués avec un polymère ou une protéine.

2. Composition pharmaceutique selon la revendication 1, dans laquelle le peptide inclut la séquence d'acides aminés suivante : VLNLCALSVDRYRAVASWSRVI ou un de ses conjugués avec un polymère ou une protéine.

3. Composition pharmaceutique selon la revendication 1, dans laquelle le peptide inclut la séquence d'acides aminés suivante CALSVDRYRAVASWGIPLITAIEI ou un de ses conjugués avec un polymère ou une protéine.

4. Composition pharmaceutique selon la revendication 1, dans laquelle le peptide inclut la séquence d'acides aminés suivante : VLNLCALSVDRYRAVASWSRVI-NH2 ou un de ses conjugués avec un polymère ou une protéine.

5. Composition pharmaceutique selon la revendication 1, dans laquelle le peptide inclut la séquence d'acides aminés suivante: VLNLCALSVDRYRAVASGSRVI-NH2 ou un de ses conjugués avec un polymère ou une protéine.

6. Composition pharmaceutique selon la revendication 1, dans laquelle le peptide inclut la séquence d'acides aminés suivante : VLNLCALSVDRYGAVASWSRVI-NH₂ ou un de ses conjugués avec un polymère ou une protéine.

7. Composition pharmaceutique selon la revendication 1, dans laquelle le peptide inclut la séquence d'acides aminés suivante : VLNLCALSVDGYRAVASWSRVI-NH2 ou un de ses conjugués avec un polymère ou une protéine.

8. Composition pharmaceutique selon la revendication 1, dans laquelle le peptide inclut la séquence d'acides aminés suivante : VLNLGALSVDRYRAVASWSRVI-NH2 ou un de ses conjugués avec un polymère ou une protéine.

9. Composition pharmaceutique selon la revendication 1, dans laquelle le peptide inclut la séquence d'acides aminés suivante : CALSVDRYRAVASW-NH2 ou un de ses conjugués avec un polymère ou une protéine.

10. Composition pharmaceutique selon la revendication 1, dans laquelle le peptide inclut la séquence d'acides aminés suivante : CALSVDRYRAVASWC-NH2 ou un de ses conjugués avec un polymère ou une protéine.

11. Composition pharmaceutique selon la revendication 1, dans laquelle le peptide inclut la séquence d'acides aminés suivante: VALSVDRYRAVASW-NH2 ou un de ses conjugués avec un polymère ou une protéine.

12. Composition pharmaceutique comprenant un inhibiteur de l'activité de l'endothéline comprenant un peptide
- incluant une partie de la séquence d'acides aminés d'un récepteur humain de l'endothéline telle qu'elle corresponde à une région avec au moins 80 % de complémentarité entre les séquences d'acides aminés, quand on compare la séquence d'acides aminés estimée sur la base d'un ADNc du récepteur humain de l'endothéline à celle estimée sur la base d'un ADNc d'un précurseur humain de l'endothéline et
- incluant une des séquences d'acides aminés (a) à (d) suivantes : 
(a) QGIGIPLITAIEI
(b) IADNAERYSANLSSHV
(c) FLGTTLRPPNLALP
(d) LNRRNGSLRIALSEHLKNRREVA
ou un de ses conjugués avec un polymère ou une protéine.

13. Inhibiteur de l'activité de l'endothéline comprenant un peptide incluant une séquence d'acides aminés choisie parmi: 
VLNLCALSVDRYRAVASWSRVI,
CALSVDRYRAVASWGIPLITAIEI,
 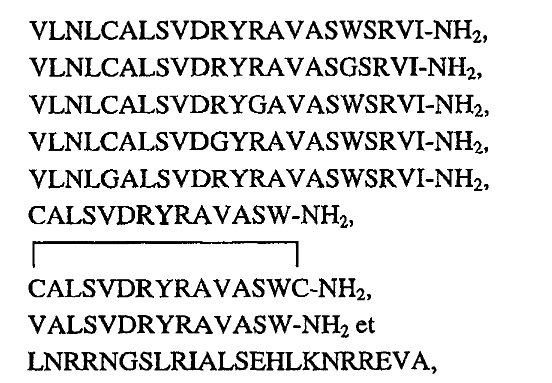
VLNLCALSVDRYRAVASWSRVI-NH2,
VLNLCALSVDRYRAVASGSRVI-NH2,
VLNLCALSVDRYGAVASWSRVI-NH2,
VLNLCALSVDGYRAVASWSRVI-NH2,
VLNLGALSVDRYRAVASWSRVI-NH2,
CALSVDRYRAVASW-NH2,
CALSVDRYRAVASWC-NH2,
VALSVDRYRAVASW-NH2 et
LNRRNGSLRIALSEHLKNRREVA,
 ou un de ses conjugués avec un polymère ou une protéine.
